# EUROPÄISCHE PATENTANMELDUNG

(11) **EP 0 573 133 A1**
(43) Veröffentlichungstag der Anmeldung: **08.12.1993**
(21) Anmeldenummer: 93250189.3
(22) Anmeldetag: 11.10.1989
(51) Int. Cl.: A61K 31/565, A61K 9/70

(54) **Mittel zur transdermalen Applikation das Gestoden enthält**

(30) Priorität: 27.10.1988 DE 3836862; 29.03.1989 DE 3910578
(62) Teilanmeldung aus: 89912449.9
(71) Anmelder: SCHERING AKTIENGESELLSCHAFT, D-13342 Berlin (DE)
(72) Erfinder: Günther, Clemens, D-1000 Berlin 21 (DE); Täuber, Ulrich, D-13359 Berlin (DE); Schmidt-Gollwitzer, Karin, D-1000 Berlin 39 (DE); Tack, Johannes-Wilhelm, D-1000 Berlin 20 (DE); Riedl, Jutta, D-1000 Berlin 21 (DE)

(57) **Zusammenfassung**

Ein Mittel zur transdermalen Applikation wird beschrieben, welches dadurch gekennzeichnet ist, daß es Gestoden gegebenenfalls in Kombination mit einem oder zwei Östrogen(en) enthält.

## Beschreibung

Die Erfindung betrifft ein Mittel zur transdermalen Applikation, dadurch gekennzeichnet, daß es Gestoden gegebenenfalls in Kombination mit einem oder mehreren Östrogen(en) enthält.

Gestoden (13-Ethyl-17β-hydroxy-18,19-dinor-17α-pregna-4,15-dien-3-on), eine Verbindung der Formel 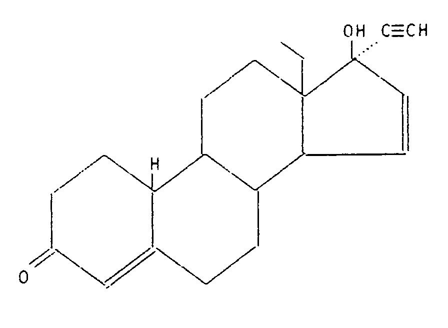
ist bekanntlich eine pharmakologisch wirksame Substanz mit einer außergewöhnlich starken gestagenen Wirksamkeit, welche in Kombination mit östrogen wirksamen Verbindungen zur Herstellung von oral zu applizierenden Mitteln konzeptionsverhindernder Wirkung (Femovan®) verwendet wird.

Es wurde nun gefunden, daß Gestoden gegebenenfalls in Kombination mit einem oder mehreren Östrogen(en) sehr gut zur Herstellung eines Mittels für die transdermale Applikation des Wirkstoffes oder Wirkstoffgemisches verwendet werden kann.

Transdermal zu applizierende Arzneimittel haben bekanntlich den Vorzug, daß sie über einen längeren Zeitraum hin eine gleichmäßigere Freisetzung des Wirkstoffs ermöglichen, als dies in der Regel bei anders - wie zum Beispiel oral -zu applizierenden Mitteln möglich ist. Diese Eigenschaften lassen sich in einer Reihe von endokrinen Erkrankungen vorteilhaft ausnutzen. Für in Wasser schwer lösliche Steroidhormone, wie zum Beispiel die Gestagene ist es aber in der Regel recht problematisch, transdermale Systeme zu erstellen, die eine zur Therapie ausreichende Penetration des Wirkstoffs durch die Haut gewährleisten.

Es wurde nun gefunden, daß es mit Hilfe des erfindungsgemäßen Mittels überraschenderweise möglich ist, eine therapeutisch ausreichende sehr gleichmäßige Penetrationsgeschwindigkeit der Steroidhormone durch die Haut zu erzielen, während dies bei den bekannten Steroidhormone enthaltenden transdermal zu applizierenden Mitteln nur bedingt möglich ist. (EP-A 137278 und EP-A 275716)
Geeignete Östrogene für das erfindungsgemäße Mittel sind beispielsweise das Estradiol, das Estriol, das Ethinylestradiol und deren Ester (EP-A 163596) wie das Estradiol-dipropionat, das Estradiol-dihexanoat und das Estradiol-didecanoat. Die erfindungsgemäßen Kombinationspräparate enthalten neben Gestoden vorzugsweise 1 bis 3 - insbesondere 1 bis 2 Östrogen(e).

Zur Herstellung pharmazeutischer Präparate kann der Wirkstoff oder das Wirkstoffgemisch in geeigneten flüchtigen Lösungsmitteln und/oder penetrationsverstärkenden Mitteln gelost oder suspendiert werden. Die erhaltenen Lösungen oder Suspensionen können mit den üblichen Hilfsstoffen, wie Matrixbildnern und Bakteriziden versetzt und gegebenenfalls nach Sterilisation in übliche Dosierbehältnisse abgefüllt werden. Andererseits ist es aber auch möglich, diese Lösungen oder Suspensionen unter Einbeziehung von Emulgatoren und Wasser zu Lotionen oder Salben weiterzuverarbeiten. Man kann auch - gegebenenfalls unter Zugabe von Treibgas - Sprays herstellen, die in den üblichen Dosierbehältnissen abgefüllt werden können.

Geeignete flüchtige Lösungsmittel sind beispielsweise niedere Alkohole, Ketone oder niedere Carbonsäureester wie Ethanol, Isopropanol, Aceton oder Ethylacetat, polare Ether, wie Tetrahydrofuran, niedere Kohlenwasserstoffe, wie Cyclohexan oder Benzin oder auch Halogenkohlenwasserstoffe, wie Dichlormethan, Trichlormethan, Trichlortrifluorethan und Trichlorfluormethan. Es bedarf keiner Erläuterung, daß auch Gemische dieser Lösungsmittel geeignet sind.

Geeignete penetrationsverstärkende Mittel sind beispielsweise Alkohole wie 1,2-Propandiol oder Benzylalkohol, gesättigte und ungesättigte Fettalkohole mit 8 bis 18 Kohlenstoffatomen, wie Laurylalkohol oder Cetylalkohol, Kohlenwasserstoffe, wie Mineralöl, gesättigte und ungesättigte Fettsäuren mit 8 bis 18 Kohlenstoffatomen, wie Stearinsäure oder Ölsäure, Fettsäureester der allgemeinen Formel

CH₃-(CH₂)ₙ-COOR

worin
n eine Zahl von 8 bis 18 und
R einen Alkylrest mit maximal 6 Kohlenstoffatomen bedeuten,
oder Dicarbonsäurediester der allgemeinen Formel

R'OCO(CH₂)ₘCOOR'

worin
m eine Zahl von 4 bis 8 und
R' jeweils einen Alkylrest mit maximal 6 Kohlenstoffatomen bedeuten,
Fettsäureester, die sich für das erfindungsgemäße Mittel eignen, sind beispielsweise solche der Laurinsäure, Myristinsäure, Stearinsäure und Palmitinsäure,wie zum Beispiel die Methylester, Ethylester, Propylester, Isopropylester, Butylester, sec.-Butylester, Isobutylester dieser Säuren. Besonders bevorzugte Ester sind solche der Myristinsäure, wie deren Methylester und insbesondere deren Isopropylester. Geeeignete Dicarbonsäurediester sind beispielsweise beispielsweise das Diisopropyladipat, Diisobutyladipat und Diisopropylsebacat. Es bedarf keiner näheren Erläuterung, das auch Gemische dieser penetrationsverstärkenden Mittel zur Herstellung des erfindungsgemäßen Mittels geeignet sind.

Die Konzentration, in welcher der Wirkstoff- oder das Wirkstoffgemisch optimalerweise in dem Lösungsmittel gelöst oder suspendiert werden, beträgt für Gestoden üblicherweise 0,01 bis 25 Gewichtsprozent. Bei den Östrogenen ist die Konzentration naturgemäß von der Art des verwendeten Wirkstoffs und der angestrebten Einzeldosis abhängig, sie muß im Einzelfalle mittels der dem Fachmann geläufigen Vorversuche, wie zum Beispiel der Bestimmung der erreichbaren Blutplasmakonzentrationen an Wirkstoff, bei ausgewählten erfindungsgemäßen Mitteln ermittelt werden. Im allgemeinen werden auch hier Wirkstoffkonzentrationen von 0,01 bis 25 Gewichtsprozent Östrogen im erfindungsgemäßen Mittel ausreichend sein. Das Gewichtsverhaltnis von Gestoden zu dem oder den Östrogen(en) liegt bei den Kombinationspräparaten bei 5:1 bis 1:10.

Die Bestimmung des Ausmaßes der Geschwindigkeit der percutanen Resorption durch die erfindungsgemäßen Mittel kann beispielsweise mittels radioaktiv markierter Steroidhormone erfolgen.

Frisch bereitete, von subcutanem Fett befreite Haut vom Abdomen haarloser Mäuse wird in eine Franz Diffusionszelle eingespannt, die als Auffangflüssigkeit isotonische Polyethylenglykol- (MG 400)-Lösung oder Phosphat-Puffer-Lösung vom pH 7 enthält. Dann gibt man 2 µl Testlösung auf die Haut und ermittelt nach 24, 48 und 72 Stunden mittels Flüssigkeits-Scintilationszählung den Gehalt der in die Auffangflüssigkeit gelangten Steroidhormons.

Getestet wurde eine 2 gewichtsprozentige Lösung von Gestoden in Isopropylmyristat (Versuch A) und Paraffin (Versuch B).

Die nachfolgende Tabelle 1 zeigt die in diesem Versuch erhaltenen Ergebnisse:

**TABELLE 1**

| Percutaner Fluß in ng Gestoden pro cm² Hautoberfläche und Stunde | | |
|---|---|---|
| Zeitintervall | Versuch A Percutaner Fluß | Versuch B Percutaner Fluß |
| 00 - 24 h | 546 | 755 |
| 24 - 48 h | 379 | 245 |
| 48 - 72 h | 287 | 100 |

Es kann ferner gezeigt werden, daß Gestoden in nicht radioaktiv markierter Form, gelöst in 1,2-Propandiol (Versuch C) oder oder in Isopropylmyristat (Versuch D) bei postmenopausalen Frauen eine ausreichende percutane Resorption zeigt.

Getestet werden 0,4 mg Gestoden, gelöst in jeweils 200 µl des entsprechenden Vehikels, die für 48 Stunden auf einer Hautfläche von 10 cm² appliziert werden.

Die nachfolgende Tabelle 2 zeigt die in diesem Versuch erhaltenen Ergebnisse:

**Tabelle 2**

| Percutaner Fluß in ng Gestoden pro cm² Hautoberfläche und Stunde | | |
|---|---|---|
| | Steady state Plasmaspiegel pg/ml | Percutaner Fluß ng/cm²/Std. bei bei 24-48 Std. |
| Versuch C | 250 | 43 |
| Versuch D | 337 | 57 |

Eine noch gleichmäßigere Applikation mit eingestellter Dosierung des Wirkstoffes oder Wirkstoffgemisches kann man erzielen wenn man den Wirkstoff oder das Gemisch in ein transdermales therapeutisches System (TTS) einbettet. Geeignete transdermale therapeutische Systeme sind solche, die man üblicherweise zur percutanen Applikation von Wirkstoffen anwendet (Yie W. Chien: "Tansdermal Controlled Systemic Medications". Marcel Dekker, Inc. , New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Reserch 1030 Hamilton Court Menlo Park CA 94025 1415) 328-2228).

So kann man beispielsweise ein solches transdermales therapeutisches System verwenden, welches aus
a) einer undurchlässigen Deckschicht,
   einer an der Deckschicht haftenden, das Gestoden,
   gegebenenfalls das oder die Östrogen(e) und gewünschtenfalls penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
   einer abziehbaren Schutzschicht, oder
b) einer undurchlässigen Deckschicht,
   einem an oder in der Deckschicht befindlichen, das Gestoden, gegebenenfalls das oder die Östrogen(e) und gewünschtenfalls penetrationsverstärkende Mittel enthaltenden Arzneimittelreservoir,
   für diese Komponenten durchlässigen Polymerschicht,
   einer durchlässigen gegebenenfalls penetrationsverstärkende Mittel enthaltende Hauthaftkleberschicht und
   einer abziehbaren Schutzschicht besteht.

Ein transdermales therapeutisches System gemäß Variante a stellt ein einfaches Matrixsystem dar. Es kann beispielsweise wie folgt hergestellt werden.

Eine Lösung oder Suspension von 1 bis 25 Gew. % Wirkstoff oder Wirkstoffgemisch, 0-40 Gew. % eines penetrationsverstärkenden Mittels, 30-70 Gew. % eines medizinisch üblichen Klebers aufgefüllt mit einem geeigneten flüchtigen Lösungsmittels zu 100 Gew. % wird auf eine plane undurchlässige Deckschicht gestrichen und nach dem Trocknen mit einer abziehbaren Schutzschicht versehen.

Verwendet man einen medizinisch üblichen Matrixbildner, der nach dem Trocknen des Systems nicht oder nicht ausreichend auf der Haut haftet, so kann man das System vor dem Aufbringen der abziehbaren Schutzschicht noch zusätzlich mit einem Hauthaftkleber abdecken oder umgeben.

Geeignete Lösungsmittel und penetrationsverstärkenden Mittel sind beispielsweise die bereits erwähnten Flüssigkeiten dieser Art. Als medizinisch übliche Kleber eignen sich beispielsweise Polyacrylate, Silicone, Polyurethane, sowie natürliche oder synthetische Kautschuke. Als weitere Matrixbildner kommen Celluloseether, Polyvinylverbindungen oder Silikate in Betracht.

Als Schutzschichten eignen sich alle Folien, die man üblicherweise bei transdermalen therapeutischen Systemen anwendet. Solche Folien sind beispielsweise silikonisiert oder fluorpolymerbeschichtet.

Als Deckschicht kann man bei diesem System beispielsweise 10 bis 100 µm dicke Folien aus Polyethylen oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise über eine Fläche von 5 bis 100 cm².

Ein transdermales therapeutisches System gemäß Variante b kann beispielsweise wie folgt hergestellt werden.

Eine undurchlässige Folie wird durch Wärme und/oder Zug so verformt, daß eine 0,1 bis 3 ml fassende Ausbuchtung entsteht. Diese wird mit einer wirkstoffhaltigen Lösung oder Suspension enthaltend 1-50 Gew. % Wirkstoff oder Wirkstoffgemisch in einem penetrationsverstärkenden Mittel gefüllt. Die wirkstoffhaltige Lösung oder Suspension kann auch mit bis zu 10 Gew. % Matrixbildner verdickt sein.

Als Abdeckung des Reservoirs zur Haut hin dient eine aufgeschweißte oder aufgeklebte durchlässige Polymerschicht, auf welche eine durchlässige Hauthaftkleberschicht und eine abziehbare Schutzschicht angebracht wird.

Es können bei diesem System die oben erwähnten penetrationsverstärkenden Mittel angewendet werden. Als durchlässige Polymerschicht wird beispielsweise eine 20 bis 200 µm dicke Folie aus Celluloseestern, Celluloseethern, Siliconen oder Polyolefinverbindungen verwendet. Durch Varitation dieser Polymerschicht läßt sich die Diffusionsgeschwindigkeit des Wirkstoffes oder Wirkstoffgemisches innerhalb weiter Grenzen variieren.

Als Kleber und Schutzschicht eignen sich die gleichen Materialien, die bei dem transdermalen therapeutischen System gemäß Variante a beschrieben sind.

So lassen sich durch einfache Variation der verschiedenen Parameter transdermale therapeutische Systeme mit unterschiedlichen Freisetzungsraten des Wirkstoffes oder Wirkstoffgemisches herstellen, die zwecks Lagerung beispielsweise in Aluminiumfolien verpackt werden können.

Es wurde bereits erwähnt, daß das erfindungsgemäße Mittel zur transdermalen Applikation auch als Lotion, Salbe oder als Spray zubereitet sein kann. Derartige Zubereitungen sind in den Fällen von Vorteil, wo keine Okklusion gewünscht wird.

Ein Emulsionsgel zur transdermalen Applikation besteht beispielsweise aus dem Wirkstoff oder Wirkstoffgemisch, penetrationsverstärkenden Mitteln, Emulgatoren (wobei ambiphile Vertreter der penetrationsverstärkenden Mittel als Emulgatoren dienen können) und gegebenenfalls Matrixbildnern. Eine typische Rezeptur besteht aus 0,1-25 Gew. % Wirkstoff oder Wirkstoffgemisch, 0-10 Gew. % Emulgator 0-5 Gew. % Matrixbildner, 0 bis 50 Gew. % penetrationsverstärkenden Mitteln und Wasser zu 100 Gew. %. Das Mittel wird in üblicher Weise emulgiert, und erforderlichenfalls mit den üblichen Antioxidantien, Konservierungsstoffen etc. versetzt.

Einphasige Gele erhält man beispielsweise durch Lösen oder Suspendieren des Wirkstoffs oder das Wirkstoffgemisches in Lösungsmitteln wie Wasser, niederen Alkoholen oder Mischungen derselben, gegebenenfalls unter Zusatz von penetrationsverstärkende Mittel und Verdicken mit Matrixbildnern.

Typische Rezepturen für solche Gele enthalten 0, 01-25 Gew. % Wirkstoff oder Wirkstoffgemisch, 1-20 Gew. % Matrixbildner, 0 bis 40 Gew. % penetrationsverstärkenden Mitteln ergänzt mit dem Lösungsmittel zu 100 Gew. % .

Auch diese Gele können gewünschtenfalls Antioxidantien, Konservierungsstoffe etc. enthalten.

Eine typische Sprayrezeptur ist beispielsweise folgende:
1-25 Gew. % Wirkstoff oder Wirkstoffgemisch, 0-20 Gew. % Matrixbildner,
0-60 Gew. % penetrationsverstärkenden Mitteln ergänzt mit Lösungsmitteln und gegebenenfalls Treibmitteln zu 100 %. Verwendet man Druckgaspackungen, so kann das Treibmittel entfallen.

Die erfindungsgemäßen gestodenhaltigen Mittel zur transdermalen Applikation können zur Behandlung der gleichen Erkrankungen angewendet werden, wie die vorbekannten, beispielsweise oral zu applizierenden Mittel die hochwirksame Gestagene enthalten. Darüberhinaus können die erfindungsgemäßen gegebenenfalls östrogenhaltigen Präparate auch zur Konzeptionsverhütung Anwendung finden. Besondere Vorteile haben die erfindungsgemäßen Mittel bei der Behandlung von Erkrankungen, die eine Langzeitbehandlung mit relativ hoher Dosierung der Wirkstoffe erfordern. Hier kann die Applikationsfrequenz wesentlich verringert werden und ein wesentlich gleichmäßiger Blutplasmaspiegel erzielt werden. Vorteilhaft ist ferner, daß gastrointestinale Nebenwirkungen nicht zu erwarten sind und bei östrogenhaltigen Kombinationspräparaten die erste Leberpassage umgangen wird und daß die Dosis an Östrogen vermindert werden kann.

Diese Vorteile lassen die östrogenfreie Monotherapeutika der vorliegenden Erfindung als besonders geeignet erscheinen um beispielsweise die Endometriose, gestagenabhängige Tumore benigne Brusterkrankungen oder das prämenstruelle Syndrom zu behandeln.

Die transdermale Anwendung von Estrogenen in sequentieller oder kontinuierlicher Kombination mit Gestoden bietet besondere Vorteile, beispielsweise zur Behandlung klimakterischer Beschwerden, zur Präventation der Osteoporose, zur Zyklusregulierung und zur Zyklusstabilisation.

Die nachfolgenden Ausführungsbeispiele dienen zur näheren Erläuterung der Erfindung. In ihnen wurden folgende Handelsprodukte verwendet:
Polyesterfolie von 0,074 mm Dicke (SkotchPak® 1009) des Herstellers 3M; Polypropylenfolie (Celgard® 2500) des Herstellers Celanese, Linerfolie SkotchPak® 1022 und 1360 vom Hersteller 3M; Transferkleber 9871 vom Hersteller 3M, Polyacrylester-Kleber vom Typ Sichello® J 6610-21 des Herstellers Henkel KG, Silikonklebstoff vom Typ X-7-2960 des Herstellers Dow Corning und Hydroxypropylcellulose des Typs Klucel® HXF des Herstellers Hercules.

### Beispiel 1

In 62,4 g einer 50 %igen Lösung von Silikonklebstoff in Benzin werden nacheinander
0,8 g Gestoden und
8,0 g 1,2-Propandiol
unter Rühren gelöst bzw. suspendiert (da die Klebstoffe trübe sind, läßt sich nicht klar entscheiden, ob eine vollständige Lösung vorliegt). Nach Entgasen des Ansatzes wird die Losung/Suspension mittels einer Beschichtungsvorrichtung auf Polyesterfolie in der Weise aufgetragen, daß nach Entfernung des flüchtigen Lösemittels ein gleichmäßiger Film von 40 g/m² Feststoffauftrag entsteht. Anschließend wird mit einem fluorpolymerbeschichteten Polyester-Liner kaschiert. Das so erhaltene Laminat wird mittels einer Stanzvorrichtung in Einzelpflaster von 10 cm² Flache geteilt und in Aluminiumfolie verpackt. Das Pflaster haftet nach Abziehen der Liner-Folie auf der Haut.

Die Gehaltsbestimmung ergibt eine gleichmäßige Wirkstoffverteilung von 0,08 mg/cm² im Mittel. Das Pflaster wird ferner über ihr in-vitro-Freigabeverhalten in Wasser über 30 Stunden bei 32° C charakterisiert. Aus einem typischen Matrixfreigabeverlauf errechnet sich nach Linearisierung eine Freisetzungsgeschwindigkeit für Gestoden von 0,4 µg/cm²/h.

### Beispiel 2

In 170 g einer 50 %igen Losung von Polyacrylester-Klebstoff in Aceton/Benzin werden nacheinander
5,0 g Gestoden und
10,0 g Isopropylmyristat
unter Rühren gelöst bzw. suspendiert. Nach Entgasen des Ansatzes wird die Lösung/Suspension mittels einer Beschichtungsvorrichtung auf Polyesterfolie in der Weise aufgetragen, daß nach Entfernung der flüchtigen Lösemittel ein gleichmäßiger Film von 100 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Flache geteilt und in Aluminiumfolie verpackt Das Pflaster haftet nach Abziehen der Liner-Folie auf der Haut.

Der Gehalt an Gestoden beträgt im Mittel 0,5 mg/cm². Die mittlere Freigabegeschwindigkeit von Gestoden liegt bei 0,3 µg/cm²/h.

### Beispiel 3

Zu 112 g einer 50 %igen Lösung von Polyacrylester-Klebstoff in Aceton/Benzin werden nacheinander
3,5 g Estradiol
3,5 g Gestoden und
7,0 g 1,2-Propandiol mit 10 % 1-Dodecanol
unter Rühren gelost bzw. suspendiert. Nach Entgasen des Ansatzes wird die Lösung/Suspension mittels einer Beschichtungsvorrichtung auf Polyesterfolie in der Weise aufgetragen, daß nach Entfernung der flüchtigen Lösemittel ein gleichmäßiger Film von 70 g/m² Feststoffauftrag entsteht. Anschließend wird mit einer silikonisierten wirkstofffreien Liner-Folie kaschiert. Das so erhaltene Laminat wird mittels einer Stanzvorrichtung in Einzelpflaster von 10 cm² Fläche geteilt und in Aluminiumfolie verpackt. Das Pflaster haftet nach Abziehen der Liner-Folie auf der Haut.

Der Gehalt an Estradiol und Gestoden liegt gleichermaßen bei je 0,35 mg/cm².

Aus diesem Pflaster werden innerhalb von 48 Stunden im in-vitro Test in Wasser von 32° C mit 0,6 µg/cm²/h für Estradiol höhere Freigaberaten erzielt als für Gestoden, wo der Wert 0,4 µg/cm²/h beträgt.

### Beispiel 4

Eine Polyesterfolie von 7,4 cm Durchmesser wird mittels Zug und Wärme so verformt, daß eine runde Ausbuchtung von 10 cm² Fläche entsteht. Diese wird mit 1 ml einer Suspension von
2,5 mg Estradiol und
2,5 mg Gestoden
in 1,2-Propandiol, welches 10 % Laurinsäure enthält, gefüllt. Eine Polypropylen- oder Celluloseacetatbutyrat-Folie wird am Rand aufgeschweißt. Je nach Druck pro Zeiteinheit liegt die Siegeltemperatur zwischen 70° C und 100° C. Auf die durchlässige Polymerschicht wird Haftkleberfolie transferiert. Das Pflaster wird mit einem Liner versehen und in Aluminiumfolie verpackt.

Aus diesem Pflaster werden für beide Wirkstoffe gleichermaßen in-vitro-Freigaberaten in Wasser von 32° C zwischen 0,02 bis 0, 08 µg/cm²/h erzielt.

### Beispiel 5

In 76,78 g Ethanol (96 Vol %ig) oder Isopropanol werden nacheinander
0,2 g Estradiol
0.02 g Gestoden
10,0 g 1,2-Propandiol und
10.0 g Isopropylmyristat
gelöst. Dann setzt man der Lösung 3 g Hydroxypropylcellulose zu und entfernt aus ihr die Luft. Nach 2 Stunden Quellzeit wird das Gel in Aluminiumtuben mit dreifacher Innenschutzlackierung abgefüllt.

Die Gehaltsbestimmung ergibt eine homogene Wirkstoffverteilung im Gel mit Werten von 95 % bei 105 % des Sollwertes.

### Beispiel 6

20,00 g Gestoden werden in 1000 g Isopropylmyristat gelöst, sterilfiltriert und unter aseptischen Bedingungen in Arzneiflaschen a 5 ml abgefüllt.

## Patentansprüche

1. Mittel zur transdermalen Applikation, dadurch gekennzeichnet, daß es Gestoden gegebenenfalls in Kombination mit einem oder mehreren Östrogen(en) enthält.

2. Mittel zur transdermalen Applikation gemäß Patentanspruch 1, dadurch gekennzeichnet, daß als Östrogen(e) Estradiol, Estriol, 17α-Ethinylestradiol oder Ester dieser Verbindungen verwendet werden.

3. Mittel zur transdermalen Applikation gemäß Patentanspruch 1 und 2, dadurch gekennzeichnet, daß das Mittel ein transdermales therapeutisches System (TTS) ist.

4. Mittel zur transdermalen Applikation gemäß Patentanspruch 3, dadurch gekennzeichnet, daß das transdermale therapeutische System aus
a ) einer undurchlässigen Deckschicht,
einer an der Deckschicht haftenden, das Gestoden,
gegebenenfalls das oder die Östrogen(e) und gewünschtenfalls penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
einer abziehbaren Schutzschicht, oder
b) einer undurchlässigen Deckschicht,
einem an oder in der Deckschicht befindlichen, das Gestoden, gegebenenfalls das oder die Östrogen(e) und gewünschtenfalls penetrationsverstärkende Mittel enthaltenden Arzneimittelreservoir,
einer für diese Komponenten durchlässigen Polymerschicht,
einer durchlässigen gegebenenfalls penetrationsverstärkende Mittel enthaltende Hauthaftkleber-Schicht und
einer abziehbaren Schutzschicht besteht.

5. Verwendung von Gestoden gegebenenfalls in Kombination mit einem oder mehreren Östrogen(en) zur Herstellung eines Mittels für die transdermale Applikation des Wirkstoffes oder Wirkstoffgemisches.

6. Verwendung von Gestoden in Kombination mit einem oder mehreren Östrogen(en) zur Herstellung eines Mittels gemäß Patentanpruch 1 , dadurch gekennzeichnet, als Östrogen(e) Estradiol, Estriol, 17α-Ethinylestradiol oder Ester dieser Verbindungen verwendet werden.

7. Verwendung von Gestoden gegebenenfalls in Kombination mit einem oder mehreren Östrogen(en) zur Herstellung eines Mittels gemäß Patentanspruch 1 und 2, dadurch gekennzeichnet, daß das Mittel ein transdermales therapeutisches System (TTS) ist.

8. Verwendung von Gestoden gegebenenfalls in Kombination mit einem oder mehreren Östrogen(en) zur Herstellung eines Mittels gemäß Patentanspruch 3, dadurch gekennzeichnet, daß das transdermale therapeutische System aus
a ) einer undurchlässigen Deckschicht,
einer an der Deckschicht haftenden, das Gestoden,
gegebenenfalls das oder die Östrogen(e) und gewünschtenfalls penetrationsverstärkende Mittel enthaltenden, für diese Komponenten durchlässigen Arzneimittelschicht, die selbsthaftend ist oder von einem Hauthaftkleber abgedeckt oder umgeben ist, welcher ebenfalls penetrationsverstärkende Mittel enthalten kann und
einer abziehbaren Schutzschicht, oder
b) einer undurchlässigen Deckschicht,
einem an oder in der Deckschicht befindlichen, das Gestoden, gegebenenfalls das oder die Östrogen(e) und gewünschtenfalls penetrationsverstärkende Mittel enthaltenden Arzneimittelreservoir,
einer für diese Komponenten durchlässigen Polymerschicht,
einer durchlässigen gegebenenfalls penetrationsverstärkende Mittel enthaltende Hauthaftkleber-Schicht und
einer abziehbaren Schutzschicht besteht.

9. Verwendung von östrogenfreien Mitteln zur transdermalen Applikation gemäß Patentanspruch 1 bis 4 zur Behandlung der Endometriose, zur Behandlung von gestagenabhangiger Tumoren und zur Behandlung des prämenstruellen Syndroms.

10. Verwendung von Mitteln zur transdermalen Applikation gemäß Patentanspruch 1 bis 4 gegebenenfalls in Kombination mit östrogenhaltigen Mitteln zur Behandlung klimakterischer Beschwerden, zur Prävention der Osteoporose, zur Zyklusregulierung und zur Zyklusstabilisation.
